# EUROPEAN PATENT SPECIFICATION

(11) **EP 1 061 925 B1**
(45) Date of publication and mention of the grant of the patent: **30.10.2002**
(21) Application number: 99909110.1
(22) Date of filing: 16.03.1999
(51) Int. Cl.: A61K 31/502, A61K 31/4196, A61K 31/14, A61K 31/465, A61K 31/445, A61P 25/28, A61P 25/16

(54) **COMBINATION OF A GABA-A ALPHA 5 INVERSE AGONIST AND A NICOTINIC AGONIST**
KOMBINATION VON EINEM INVERSEN AGONISTEN DES GABA-A ALPHA-5 REZEPTOR SUBTYPS UND NICOTINERGE AGONISTEN
COMBINAISON D'UN AGONISTE INVERSE DU GABAa ALPHA 5 ET D'UN AGONISTE DE LA NICOTINE

(30) Priority: 16.03.1998 GB 9805559
(43) Date of publication of application: 27.12.2000
(73) Proprietor: MERCK SHARP & DOHME LTD., Hoddesdon Hertfordshire EN11 9NU (GB)
(72) Inventor: DAWSON, Gerard Raphael, Harlow, Essex CM20 2QR (GB)
(74) Representative: Horgan, James Michael Frederic
(86) International application number: GB9900800
(87) International publication number: WO99047142

(56) References cited:
- EP-A- 0 377 520
- WO-A-96/25948
- WO-A-98/04560
- ROSENBERG D R ET AL: "COGNITIVE ENHANCING AGENTS FOR THE TREATMENT OF SENILE DEMENTIA OF THE ALZHEIMER'S TYPE" DRUGS OF TODAY / MEDICAMENTOS DE ACTUALIDAD, vol. 26, no. 7, 1 October 1990 (1990-10-01), pages 449-471, XP000616601 ISSN: 0025-7656

## Description

The present invention relates to a combination of a nicotinic agonist and an inverse agonist of the GABA_{A} α₅ receptor subtype as detailed below and the use of the combination in treating neurodegenerative conditions such as Alzheimer's Disease.

Alzheimer's Disease is a poorly understood neurodegenerative condition mainly affecting the elderly but also younger people who are generally genetically predispositioned to it.

One postulated method of treatment comprises the administration of nicotinic agonists which act on the cholinergic system. However this method suffers from the disadvantages that these compounds induce a range of side-effects including diarrhoea, salivation and nausea.

The present invention provides a new and surprisingly effective synergistic combination of an nicotinic agonist and an inverse agonist of the GABA_{A} α₅ receptor subtype, as detailed below, for separate, sequential or simultaneous administration.

The present invention provides a greater than expected improvement in the condition of subjects suffering from a neurodegenerative with an associated cognitive deficit, such as Alzheimer's Disease or Parkinson's disease, or from a cognitive deficit which may arise from a normal process such as aging or from an abnormal process such as injury, than would be expected from administration of the active ingredients alone. Further, the combination allows for a lower overall dose of each of the active ingredients to be administered thus reducing side effects and decreasing any reduction in the effectiveness of each of the active ingredients over time.

Nicotinic agonists which may be used include any which are known to the skilled person. Examples are nicotine, lobeline, tetramethylammonium, 1,1-dimethyl-4-phenylpyrazinium and ABT 418. EP-A-0 377 520 discloses nicotine can be used to enhance cognition in Alzheimer's disease.

WO-A-9 804 560 discloses substituted triazolo pyridazine derivatives as GABA-A α5 receptor subtype inverse agonists for enhancing cognition.

Thus the inverse agonist has a functional efficacy at the α₅ receptor subunit of less than -20% and functional efficacies at the α₁, α₂, and α₃ receptor subunits of between -20 and +20%. By functional efficacy is meant the percentage modulation of the EC₂₀ response produced by GABA, upon coadministration of the inverse agonist, in oocytes expressing GABA_{A} receptor channels containing the α receptor subunit under test. Details of this measurement are given in WO-A-9625948.

The inverse agonist binds selectively to GABA_{A} receptors containing the α₅ subunit 10, 25 and particularly 50 times compared to GABA_{A} receptors subunits containing the α₁, α₂ or α₃ subunits. This binding selectivity is shown over all these subunits.

The inverse agonist, which is disclosed in WO-A-9850385, namely:
3-(5-methylisoxazol-3-yl)-6-(1-methyl-1,2,3-triazol-4-yl)methyloxy-1,2,4-triazolo[3,4-a]phthalazine, is the inverse agonist of choice for the present invention.

The present invention also provides a pharmaceutical composition comprising a nicotinic agonist, the above-mentioned inverse agonist of the GABA_{A} α₅ receptor subtype and a pharmaceutically acceptable carrier.

There is also provided a kit of parts comprising a first pharmaceutical composition comprising a nicotinic agonist and a first pharmaceutically acceptable carrier and a second pharmaceutical composition comprising the mentioned inverse agonist of the GABA_{A} α₅ receptor subtype and a second pharmaceutically acceptable carrier for simultaneous, sequential or separate administration.

There is further provided a combination of a nicotinic agonist and the mentioned inverse agonist of the GABA_{A} α₅ receptor subtype for use in a method of treatment of the human body, particularly for the treatment of a neurodegenerative disorder with associated cognitive deficit such as Alzheimer's Disease or Parkinson's disease, or of a cognitive deficit arising from a normal process such as aging or of an abnormal process such as injury. The combination is particularly beneficial in the treatment of Alzheimer's Disease.

There is also provided the use of a combination of a nicotinic agonist and the mentioned inverse agonist of the GABA_{A} α₅ receptor subtype in the manufacture of a medicament for the treatment of a neurodegenerative disorder such as Alzheimer's Disease or Parkinson's disease, or of a cognitive deficit arising from a normal process such as aging or of an abnormal process such as injury. The treatment of Alzheimer's Disease is particularly preferred.

The pharmaceutical compositions of the present invention are preferably in unit dosage forms such as tablets, pills, capsules, powders, granules, sterile parenteral solutions or suspensions, metered aerosol or liquid sprays, drops, ampoules, transdermal patches, auto-injector devices or suppositories; for oral, parenteral, intranasal, sublingual or rectal administration, or for administration by inhalation or insufflation. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums or surfactants such as sorbitan monooleate, polyethylene glycol, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of each active ingredient of the present invention. Typical unit dosage forms contain from 1 to 100 mg, for example 1, 2, 5, 10, 25, 50 or 100 mg, of each active ingredient. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatin.

For the treatment of a neurodegenerative condition, a suitable dosage level is about 0.01 to 250 mg/kg per day, preferably about 0.01 to 100 mg/kg per day, and especially about 0.01 to 5 mg/kg of body weight per day of each active ingredient. The compounds may be administered on a regimen of 1 to 4 times per day. In some cases, however, dosage outside these limits may be used.

The synergistic effect of the combination of the present invention can be shown, for example, by comparing the combined dosage of the combination with dosages of the same amount of each of the active ingredients separately on subjects using the Mini-Mental State Examination (MMSE) as described in Folstein and Folstein J. Psychiat. Res., 1975, 12, 189-198 or a variant thereof as discussed in Tombaugh and McIntyre, JAGS, 1992, 40, 922-935.

## Claims

1. A combination of a nicotinic agonist and 3-(5-methylisoxazol-3-yl)-6-(1-methyl-1,2,3-triazol-4-yl)methyloxy-1,2,4-triazolo[3,4-a]phthalazine suitable for separate, sequential or simultaneous administration.

2. A combination according to claim 1 wherein the nicotinic agonist is selected from nicotine, lobeline, tetramethylammonium, 1,1-dimethyl-4-phenylpyrazinium and ABT 418.

3. A pharmaceutical composition comprising a combination as defined in claim 1 or 2 and a pharmaceutically acceptable carrier for simultaneous administration.

4. A kit of parts comprising a first pharmaceutical composition comprising a nicotinic agonist and a first pharmaceutically acceptable carrier and a second pharmaceutical composition comprising 3-(5-methylisoxazol-3-yl)-6-(1-methyl-1,2,3-triazol-4-yl)methyloxy-1,2,4-triazolo[3,4-a]phthalazine and a second pharmaceutically acceptable carrier suitable for simultaneous, separate or sequential administration.

## Patentansprüche

1. Eine Kombination aus einem nikotinischen Agonisten und 3-(5-Methylisoxazol-3-yl)-6-(1-methyl-1,2,3-triazol-4-yl)methyloxy-1,2,4-triazol[3,4-a]phthalazin, die sich zur getrennten, aufeinanderfolgenden oder gleichzeitigen Verabreichung eignet.

2. Eine Kombination gemäß Anspruch 1, wobei der nikotinische Agonist ausgewählt ist aus Nikotin, Lobelin, Tetramethylammonium, 1,1-Dimethyl-4-phenylpyrazinium und ABT 418.

3. Eine pharmazeutische Zusammensetzung, die eine wie in Anspruch 1 oder 2 definierte Kombination und einen pharmazeutisch annehmbaren Träger zur gleichzeitigen Verabreichung enthält.

4. Ein Teilekit, der eine erste pharmazeutische Zusammensetzung, die einen nikotinischen Agonisten und einen ersten pharmazeutisch annehmbaren Träger enthält, und eine zweite pharmazeutische Zusammensetzung, die 3-(5-Methylisoxazol-3-yl)-6-(1-methyl-1,2,3-triazol-4-yl)methyloxy-1,2,4-triazol[3,4-a]phthalazin und einen zweiten pharmazeutisch annehmbaren Träger enthält, enthält und sich zur gleichzeitigen, getrennten oder aufeinanderfolgenden Verabreichung eignet.

## Revendications

1. Combinaison d'agoniste de la nicotine et de 3-(5-méthylisoxazol-3-yl)-6-(1-méthyl-1,2,3-triazol-4-yl) méthyloxy-1,2,4-triazolo[3,4-a] phtalazine, adéquate pour l'administration séparée, séquentielle ou simultanée.

2. Combinaison selon la revendication 1, dans laquelle l'agoniste de la nicotine est choisi parmi la nicotine, la lobéline, le tétraméthylammonium, le 1,1-diméthyl-4-phénylpyrazinium et l'ABT 418.

3. Composition pharmaceutique comprenant une combinaison telle que définie dans la revendication 1 ou 2, et un vecteur pharmaceutiquement acceptable pour une administration simultanée.

4. Kit d'éléments contenant une première composition pharmaceutique comprenant un agoniste de la nicotine et un premier vecteur pharmaceutiquement acceptable, et une seconde composition pharmaceutique comprenant la 3-(5-méthylisoxazol-3-yl)-6-(1-méthyl-1,2,3-triazol-4-yl) méthyloxy-1,2,4-triazolo[3,4-a] phtalazine et un second vecteur pharmaceutiquement acceptable, adéquate pour une administration simultanée, séparée ou séquentielle.
